# EUROPEAN PATENT APPLICATION

(11) **EP 4 335 433 A1**
(43) Date of publication of application: **13.03.2024**
(21) Application number: 23723104.8
(22) Date of filing: 06.03.2023
(51) Int. Cl.: A61K 9/107, A61K 47/28, A61K 47/26, A61K 9/08, A61K 9/00, A61K 38/29, A61P 19/10

(54) **ORAL PHARMACEUTICAL COMPOSITION CONTAINING TERIPARATIDE FOR PREVENTION OR TREATMENT OF OSTEOPOROSIS AND PREPARATION METHOD THEREFOR**

(30) Priority: 22.07.2022 KR 20220090893
(71) Applicant: Icure BNP Co., Ltd., Chungcheongbuk-do 27643 (KR)
(72) Inventor: CHANG, Kwan Young, Seoul 07985 (KR); KANG, Seo Hee, Seoul 07985 (KR); SEO, Yu Seon, Seoul 07985 (KR); PARK, Jin Woo, Seoul 07525 (KR)
(74) Representative: Franke, Dirk
(86) International application number: PCT/KR2023/003019
(87) International publication number: WO 2024/019246

(57) **Abstract**

The present disclosure relates to an oral pharmaceutical composition for the prevention and treatment of osteoporosis comprising an ion-binding complex including a teriparatide, an L-lysine-linked deoxycholic acid, a deoxycholic acid, and an alkyl glycoside-based surfactant. According to the present disclosure, the ion-binding complex including teriparatide according to the present disclosure can maintain the drug at a high dose by inhibiting its degradation by peptidases in the gastrointestinal tract due to the incorporation of teriparatide into the micellar structure and can improve the permeability of the drug due to the availability of various transmembrane absorption routes, which ultimately increases the oral bioavailability of the drug, teriparatide.

## Description

### Technical Field

The present disclosure was made through a total of two national research and development projects, one of which was supported by the Ministry of SMEs and Startups of the Republic of Korea, task identification number 1425143048 (assignment number: S2883658), the management agency for the above project is "Small and Medium Business Technology Information Promotion Agency", the name of the research business project is "Small and Medium Business Technology Innovation and Development (R&D)", and the name of the research project is "Development of oral teriparatide drug through enhancement of intestinal mucosal absorption of drugs", the project executing agency is "ICure B&P Co., Ltd.", and the research period is 2020.06.01 to 2022.05.31. The other was supported by the Ministry of Science and ICT of the Republic of Korea under project number 1711172975 (project number: 2022R1A5A8033794). The management organization of the above project is "Korea Research Foundation", the name of the research business project is "Regional Innovation Leading Research Center", the name of the research project is "Bio Medicine Advanced Formulation Research Center", and the project executing agency is "Mokpo University ", and the research period is 2022.06.01 to 2029.02.28.

The present disclosure relates to an oral pharmaceutical composition for the prevention or treatment of osteoporosis containing teriparatide and a method for preparing the same. More specifically, the present disclosure relates to an oral pharmaceutical composition for the prevention or treatment of osteoporosis, including an ion-binding complex composed of at least one of teriparatide, L-lysine-linked deoxycholic acid, and deoxycholic acid, and alkyl glycoside-based surfactants.

### Background Art

Current treatments for osteoporosis are primarily based on the inhibition of bone resorption by bisphosphonates to reduce bone turnover and prevent further bone loss by inhibiting bone resorption. These drugs can increase bone mineral density (BMD) and preserve existing skeletal microarchitecture by interfering with osteoclast bone resorption, but their effectiveness is limited to a short-term reduction in bone resorption and a partial reduction in fracture risk. These therapies are unable to restore the normal bone structure, potentially leading to poor bone quality, loss of bone strength, and fracture propensity.

Many patients with osteoporosis who require treatment have already lost significant amounts of bone, and there is a need to develop therapies that can increase bone mass by stimulating new bone formation. Human parathyroid hormone (hPTH), a potential candidate for anabolic therapy in osteoporosis, has attracted much attention on the basis of reports that intermittent subcutaneous (SC) administration of recombinant hPTH (rhPTH) exerts a potent anabolic effect on the skeleton and increases the rate of bone remodeling. The rate of bone remodeling results in a positive remodeling balance, leading to thicker bone. Thus, anabolic hPTH treatment can induce new bone formation through increased bone modeling and remodeling and has several advantages over antiresorptive treatment.

Two forms of rhPTH are currently approved for the management of osteoporosis in postmenopausal women and men: teriparatide [TRP; rhPTH with the first N-terminal 34 amino acids, rhPTH(1-34)] and the intact 84-amino acid form, rhPTH(1-84). However, TRP is currently only available in the form of 20 µg once daily (Forteo^{®}) or 56.5 µg once daily (Teribone^{™}) SC injections, which is inconvenient for long-term use due to delayed absorption and poor compliance with therapies required for chronic diseases. Therefore, there is a need to develop an oral TRP formulation with a therapeutic potential equivalent to the SC injectable formulation that exhibits better gastrointestinal (GI) permeation and oral absorption and may increase patient compliance. However, most peptide drugs, including TRP, have poor oral bioavailability due to peptidase sensitivity and low intestinal permeability. In addition, oral delivery of TRP presents several challenges, such as the need for a pulsatile pharmacokinetic profile to achieve the osteoanabolic activity. Therefore, the duration of drug exposure should be above the baseline level of TRP for at least 1 hour but not exceed 5 hours.

In order to solve this problem, various strategies have been investigated in the prior art, including formulation with permeation enhancers and/or co-administration with protease inhibitors, liposomes, micelles, self-micro emulsifying drug delivery systems, solid lipid nanoparticles, and encapsulation within solid matrix nano- or microparticles. Enteric, mucoadhesive, or colon-targeted coating systems have also been introduced to release peptides, and research has also been conducted to include penetration enhancers and other additives in formulations targeting targeted areas of the GI tract to minimize metabolic degradation. In addition, Guo et al. prepared an rhPTH(1-34) water-in-oil microemulsion that delayed the enzymatic degradation of rhPTH(1-34) and improved its relative oral bioavailability to 5.4% in rats, and Emisphere Technologies, Inc., used N-(5-chlorosalicyloyl)-8-aminocaprylic acid (5-CNAC) as a permeation enhancer to improve the oral absorption of TRP, which resulted in oral bioavailability of 2.1% in monkeys and progressed to clinical evaluation of oral TRP delivery. However, the development of oral TRP-5-CNAC was discontinued because it did not meet expectations with respect to anti-osteoporotic effects.

Oral peptide delivery requires a combination of delivery approaches that limit metabolic degradation as well as intestinal penetration enhancers, namely peptidases. It is important that innovative formulation strategies optimize epithelial drug release to enhance the rate and extent of drug entry, shorten GI residence time to limit exposure to peptidases, and allow penetration enhancer additives to achieve the critical concentrations needed to promote flow. Leading permeation enhancer-based technologies appear to provide inhibition of peptidases by non-covalent complexation of the peptide with the carrier, and the most promising strategies to limit degradation include hydrophobizing the permeation enhancer, structural modification (e.g., amino acid substitution and N-terminal amidation), and nanoencapsulation.

Most permeation enhancers in oral delivery systems are relatively mild surfactant-based detergents, including C10, ethylenediaminetetraacetic acid fatty acids, acylcarnitines, acylated amino acids, alkyl polyethoxylates, glyceryl polyethoxylates, channel-forming peptides, bile salts, glycerides, sucrose esters, polysorbates, enamines, and salicylates. Since these permeation enhancers non-specifically perturb the intestinal mucosa by opening tight junctions (TJs) or by altering the integrity of epithelial cell membranes and increasing their fluidity, there is a need for formulations to improve cellular permeation while minimizing the use of permeation enhancers.

Conventionally, techniques for increasing the permeability and bioavailability of teriparatide have been developed. Danish Patent Application Laid-open Publication No. 01643978 describes a conventional enteric-coated tablet that allows PTH to be released 2 hours after oral administration to minimize degradation of the drug in the gastrointestinal tract, thereby improving absorption. However, the low oral bioavailability of PTH is mainly due to the degradation of the drug in the gastrointestinal tract, as well as the low lipid affinity of the drug molecule and the low permeability of the intestinal membrane itself due to the drug molecule's large molecular weight. Therefore, conventional enteric-coated oral dosage forms are insufficient to achieve sufficient oral bioavailability to exert the therapeutic effects of PTH. In addition, Korean Patent Application Laid-open Publication No. 2017-0125793 describes combining a therapeutic agent with a cationic conjugate to prepare a core complex, covalently bonding a bile acid to an anionic polymer, and electrostatically coupling the cationic conjugate to the anionic conjugate to enable the therapeutic agent to be absorbed by the patient through a bile acid transporter in the gastrointestinal tract and enter the enterohepatic circulation, thereby improving patient compliance. However, there is no disclosure in the literature of a composition utilizing n-dodecyl-β-D-maltoside as an alkyl glycoside-based surfactant to increase the bioavailability of teriparatide, as described in the present disclosure.

Accordingly, the present inventors have made good faith efforts to overcome the above problems of the related art and have discovered that ion-binding complexes including one or more of teriparatide (TRP), L-lysine-linked deoxycholic acid, deoxycholic acid, and an alkyl glycoside-based surfactant, it has been found that the therapeutic effect of osteoporosis can be significantly increased by improving the intestinal membrane permeability and oral bioavailability of TRP, thus completing the present disclosure.

### Disclosure

### Technical Problem

Therefore, the main objective of the present disclosure is to provide an oral pharmaceutical composition for the prevention or treatment of osteoporosis including teriparatide, which can significantly increase the therapeutic effectiveness of osteoporosis by improving the intestinal membrane permeability and oral bioavailability of teriparatide.

Another objective of the present disclosure is to provide a process for preparing an oral pharmaceutical composition for the prevention or treatment of osteoporosis including the teriparatide.

### Technical Solution

According to one aspect of the present disclosure, the present disclosure provides an oral pharmaceutical composition for the prevention and treatment of osteoporosis including ion-binding complexes composed of at least one of teriparatide (TRP), L-lysine-linked deoxycholic acid, and deoxycholic acid, and an alkyl glycoside-based surfactant.

Human parathyroid hormone [rhPTH (1-34); teriparatide (TRP)] is a drug used to manage osteoporosis, and the current formulation of teriparatide therapy is an injectable, which has been associated with problems such as delayed drug absorption and low compliance. Accordingly, the inventors of the present disclosure attempted to increase patient compliance by formulating a therapeutic formulation of teriparatide into an oral formulation and to increase oral bioavailability by improving the drug permeability of the oral formulation.

As used in the present disclosure, the term "ion-binding complex" refers to a material in which the hydrophobic region of the complex formed by binding lysine-linked deoxycholic acid to acidic amino acids of teriparatide and binding deoxycholic acid to basic amino acids is incorporated into the micelle structure by interacting with the alkyl glycoside-based surfactant.

Teriparatide is a peptide-based drug, and its high sensitivity to peptidase, low membrane permeability due to high molecular weight, and hydrophilicity is the cause of lowering oral bioavailability. Therefore, bioavailability can be improved by increasing the lipophilicity of the peptide through charge relaxation of the teriparatide chain to enhance membrane transport of the teriparatide and by incorporating the teriparatide into a micellar structure to prevent degradation by peptidases to increase the permeability of high doses of the drug. The amino acid sequence that makes up the peptide of a teriparatide has a total of four acidic (negatively charged) amino acids, aspartic acid, and glutamic acid, and a total of eight basic (positively charged) amino acids, arginine, histidine, and lysine. Therefore, in the present disclosure, the charge of the teriparatide chain is relaxed by combining the acidic amino acid with a positively charged lysine-linked deoxycholic acid and the basic amino acid with a negatively charged deoxycholic acid. Incorporating the teriparatide, lysine-linked deoxycholic acid, and deoxycholic acid (TRP/LDA/DA) complex into an alkyl glycoside-based surfactant, n-dodecyl-β-D-maltoside (DM), to form a micellar structure, which enhanced membrane transport in addition to protecting the teriparatide from peptidases.

In the oral pharmaceutical composition of the present disclosure, the alkyl glycoside-based surfactant may be any alkyl glycoside-based surfactant selected from the group consisting of decyl glucoside, arachidyl glucoside, butyl glucoside, C10 to 16 alkyl glucoside, C12 to 18 alkyl glucoside, C12 to 20 alkyl glucoside, C20 to 22 alkyl glucoside, caprylyl/capryl glucoside, caprylyl glucoside, cetearyl glucoside, coco-glucoside, ethyl glucoside, hexadecyl D-glucoside, isostearyl glucoside, lauryl glucoside, myristyl glucoside, octadecyl D-glucoside, octyldodecyl glucoside, and undecyl glucoside. Preferably, the surfactant is characterized in that it is at least one of n-dodecyl-β-D-maltoside and n-Octyl-β-D-glucopyranoside, more preferably n-dodecyl-β-D-maltoside.

Polyoxyethylene-based surfactants, which are conventionally used to enhance the permeability of drugs, frequently suffer from degradation or denaturation of peptide drugs and protein drugs during manufacturing and storage due to the spontaneous oxidation of the ether bonds and unsaturated alkyl chains contained in the polyoxyethylene moiety, resulting in the generation of chemically reactive species such as peroxides, epoxy acids, and aldehydes. Accordingly, the present inventors selected alkyl glycoside-based surfactants as surfactants that can enhance the permeability of drugs without damaging and denaturing peptide drugs and protein drugs and sought to utilize them in the oral pharmaceutical compositions of the present disclosure.

In the oral pharmaceutical compositions of the present disclosure, the lysine-linked deoxycholic acid may be included from 1 to 7 moles per mole of teriparatide, preferably from 1 to 5 moles, most preferably 5 moles. When less than 1 mole of lysine-linked deoxycholic acid is included, less than 1 mole of lysine-linked deoxycholic acid is bound to 1 molecule of teriparatide, resulting in the formation of a complex that is not effective in enhancing intestinal membrane permeability, and when more than 7 moles are included, irreversible precipitation of teriparatide may occur.

In the oral pharmaceutical compositions of the present disclosure, the deoxycholic acid may be included from 1 to 15 moles per mole of teriparatide, preferably from 2 to 15 moles, and most preferably from 7 moles. When less than 1 mole of deoxycholic acid is included, less than 1 mole of deoxycholic acid is bound to 1 molecule of teriparatide, and the formation of the complex sought to be implemented in the present disclosure is not achieved, resulting in insufficient effectiveness in enhancing intestinal membrane permeability, and when more than 15 moles are included, irreversible precipitation of teriparatide may occur, resulting in low drug content.

In the oral pharmaceutical composition of the present disclosure, the alkyl glycoside-based surfactant may be included in a 1:1 to 1:50 weight ratio to the teriparatide, preferably in a 1:5 to 1:20 weight ratio, and most preferably in a 1:15 weight ratio. When the alkyl glycoside surfactant is less than 1:1 by weight, it is difficult to improve the permeability of the teriparatide, and when the alkyl glycoside surfactant is more than 1:50, it may cause side effects such as irritation of the gastrointestinal mucosa.

In the present disclosure, various experiments have confirmed the effect of improving the permeability of ion-binding complexes and improving the oral bioavailability of teriparatide according to various combinations and ratios of at least one of teriparatide, L-lysine-linked deoxycholic acid, and deoxycholic acid with alkyl glycoside-based surfactants.

Specifically, when the in vitro artificial intestinal membrane permeability of teriparatide alone (Comparative Example 1) and teriparatide bound to lysine-linked deoxycholic acid or deoxycholic acid (Comparative Examples 2 and 3, respectively) was compared, it was found that the permeability was enhanced due to the increased lipophilicity of teriparatide by complex formation, suggesting that charge relaxation of the peptide chain can enhance membrane transport of teriparatide via passive diffusion. Furthermore, it was found that the permeability of the teriparatide incorporated into the alkyl glycoside-based surfactant (Comparative Examples 6, 7) was significantly increased compared to that of the teriparatide not incorporated into the alkyl glycoside-based surfactant (Comparative Example 1), and it can be seen from these results that the lipophilicity is further enhanced by the alkyl glycoside-based surfactant to maximize the permeation of the teriparatide (see Experimental Example 2). Accordingly, the present inventors have derived compositions that further include an alkyl glycoside-based surfactant for improved permeability of the teriparatide, lysine-linked deoxycholic acid, and deoxycholic acid complexes.

Furthermore, it was found that the ion-binding complexes, according to the present disclosure (Example 1, TRP/LDA/DA-DM), exhibited higher cellular uptake than the complexes not including alkyl glycoside-based surfactants (Example 5, TRP/LDA/DA) (see Experimental Example 3). These results further confirm the potential for alkyl glycoside-based surfactants to enhance drug permeability by altering barrier properties and participating in additional permeation mechanisms including ASBT-mediated transport, clathrin/caveola-mediated endocytosis and/or macropinocytosis. As a result of confirming cell penetration by ASBT-mediated transport among the membrane penetration mechanisms, cellular uptake of the ion-binding complexes according to the present disclosure (Example 1, TRP/LDA/DA-DM) was significantly increased in ASBT-expressing cells compared to ASBT- non-expressing cells (see Experimental Example 3), suggesting that the ion-binding complexes according to the present disclosure may exert synergistic effects on drug permeability through involvement in ASBT-mediated transport.

Furthermore, to confirm that the ion-binding complexes according to the present disclosure (Example 1, TRP/LDA/DA-DM) enhance drug permeability not only through ASBT-mediated transport but also through various intercellular uptake pathways, the transport mechanisms of teriparatide were identified and found to utilize clathrin-mediated endocytosis, caveola-mediated endocytosis, macropinocytosis, and ER/Golgi pathways (see Experimental Example 4). These results suggest that the ion-binding complexes, according to the present disclosure, are capable of membrane migration through a variety of pathways, thereby exhibiting synergistic effects on drug permeability.

Furthermore, to confirm that the ion-binding complexes, according to the present disclosure (Example 1, TRP/LDA/DA-DM), have the same effect in vivo as in vitro, the oral absorption of the drug in mice was checked. As a result, a significant increase in oral bioavailability was observed when compared to oral administration of teriparatide alone (Experimental Example 6), suggesting that intestinal membrane permeability and absorption of teriparatide in mice can be achieved by incorporating teriparatide, lysine-linked deoxycholic acid and deoxycholic acid complexes into micelle structures due to interaction with alkyl glycoside-based surfactants

According to another aspect of the disclosure, the disclosure provides a method of preparing an oral pharmaceutical composition for the prevention and treatment of osteoporosis, the method including: a first step of preparing a teriparatide solution, a deoxycholic acid solution, a lysine-linked deoxycholic acid solution, and an alkyl glycoside-based surfactant solution, respectively; a second step of mixing the alkyl glycoside-based surfactant solution into the teriparatide solution prepared in the first step; a third step of mixing a lysine-linked deoxycholic acid solution into the solution mixed in the second step; and a fourth step of mixing the deoxycholic acid solution into the solution mixed in the third step. The order of adding the lysine-bonded deoxycholic acid solution and the deoxycholic acid solution in the third and fourth steps of the above method of preparation is not necessarily mean that the lysine-bonded deoxycholic acid solution is added after the deoxycholic acid solution is added, and the order of mixing the two components can be changed according to convenience during the preparation process, but it is most preferable to add the components added in a relatively large molar ratio later so that sufficient binding of the teriparatide to the lysine-bonded deoxycholic acid and deoxycholic acid can be achieved.

In the method of preparing the oral pharmaceutical composition of the present disclosure, the pH of the teriparatide solution, the deoxycholic acid solution, and the lysine-linked deoxycholic acid solution is in a range of 3 to 8, preferably pH 4. Since teriparatide is most stable under conditions of pH 4 and exhibits the most positive and negative charges at the same time, the above range of pH is most suitable because it can maximize the molar ratio of ionic binding of lysine-linked deoxycholic acid and deoxycholic acid, and therefore it is difficult to maximize the ionic binding of teriparatide to deoxycholic acid and lysine-linked deoxycholic acid when the pH is out of the pH range.

In the method of preparing the oral pharmaceutical composition of the present disclosure, the alkyl glycoside-based surfactant in the second step may be included in a 1:1 to 1:50 weight ratio with respect to the teriparatide, preferably in a 1:5 to 1:20 weight ratio, most preferably in a 1:15 weight ratio. When the alkyl glycoside surfactant is less than 1:1 by weight, it is difficult to improve the permeability of the teriparatide, and when the alkyl glycoside surfactant is more than 1:50 by weight, it may cause side effects such as irritation of the gastrointestinal mucosa.

In the method of preparing the oral pharmaceutical composition of the present disclosure, the lysine-linked deoxycholic acid in the third step may be included from 1 to 7 moles per mole of teriparatide, preferably from 1 to 5 moles, most preferably 5 moles. When less than 1 mole of lysine-linked deoxycholic acid is included, less than 1 mole of lysine-linked deoxycholic acid is bound to 1 molecule of teriparatide, resulting in the formation of a complex that is not effective in enhancing intestinal membrane permeability, and when more than 7 moles of lysine-linked deoxycholic acid are included, irreversible precipitation of teriparatide may occur.

In the method of the oral pharmaceutical compositions of the present disclosure, the deoxycholic acid may be included from 1 to 15 moles per mole of teriparatide, preferably from 2 to 15 moles, and most preferably from 7 moles. When less than 1 mole of deoxycholic acid is included, less than 1 mole of deoxycholic acid is bound to 1 molecule of teriparatide, and the formation of the complex sought to be implemented in the present disclosure is not achieved, resulting in insufficient effectiveness in enhancing intestinal membrane permeability, and when more than 15 moles of deoxycholic acid are included, irreversible precipitation of teriparatide may occur, resulting in low drug content.

The method of preparing the oral pharmaceutical composition of the present disclosure further includes adding mannitol and sucrose to the mixture of the fourth step, followed by a drying step using a method such as a spray drying, freeze drying, or the like.

The ion-binding complexes prepared according to the above methods may be prepared in the form of an oral solid dosage form selected from the group consisting of powders, granules, pellets, tablets, and capsules. For example, the pharmaceutical composition of the disclosure in the form of a capsule may be prepared by filling a capsule with a powder or granule obtained by mixing the dry ion-binding complexes with a binder, disintegrating agent, diluent, lubricants, and pharmaceutically acceptable additives. The pharmaceutical composition of the present disclosure in the form of tablets or pellets may be prepared by pressing a powder or granule obtained by mixing a dry ion-binding complex with a binder, disintegrating agent, diluent, lubricant, and pharmaceutically acceptable additives.

The binder used to prepare the ion-binding complex according to the present disclosure in the form of powders, granules, capsules, pellets, and tablets may be any binders conventionally used to prepare powders, granules, capsules, pellets, and tablets and may include, for example, at least one selected from the group consisting of polyvinyl pyrrolidone, gelatin, starch, sucrose, methyl cellulose, ethyl cellulose, hydroxypropyl cellulose, hydroxypropyl alkyl cellulose but is not limited thereto. In addition, as the disintegrating agent, any disintegrating agent conventionally used to prepare granules, capsules, and tablets may be used, and the disintegrating agent may include, for example, at least one selected from the group consisting of cross-linked polyvinyl pyrrolidone, cross-linked sodium carboxymethyl cellulose, cross-linked calcium carboxymethyl cellulose, cross-linked carboxymethyl cellulose, sodium starch glycolate, carboxymethyl starch, sodium carboxymethyl starch, potassium methacrylate-divinyl benzene copolymer, amylose, cross-linked amylose, starch derivative, microcrystalline cellulose, cellulose derivative, cyclodextrin, and dextrin derivatives but is not limited thereto. As the diluent, any diluent conventionally used to prepare powders, granules, capsules, pellets, and tablets may be used, and the diluent may include, for example, at least one selected from the group consisting of lactose, dextrin, starch, and microcrystalline cellulose, calcium hydrogen phosphate, anhydrous calcium hydrogen phosphate, calcium carbonate, and saccharide but is not limited thereto. As the lubricant, any lubricant conventionally used to prepare powders, granules, capsules, pellets, and tablets may be used, and the lubricant may include, for example, at least one selected from the group consisting of stearic acid, zinc stearate, magnesium stearate, calcium stearate, and talc but is not limited thereto.

The method of preparing the oral pharmaceutical composition of the present disclosure can coat the granules, capsules, tablets, and pellets prepared herein with an enteric-soluble substance and can form a coating layer on the surface of the granules, capsules, tablets, and pellets to inhibit the release of the drug under acidic conditions in the stomach after oral administration, thereby minimizing the degradation of the drug.

The enteric-soluble substance may be any material generally used to prepare an enteric-soluble dosage and may include, for example, at least one selected from the group consisting of Eudragit (methacrylic acid-ethyl acrylate copolymer), hydroxypropyl methylcellulose phthalate, acetyl succinate hydroxyl propyl methylcellulose, cellulose acetate phthalates, polyvinyl acetate phthalates, carboxy methyl ethyl cellulose, and shellac but is not limited thereto.

In addition, the enteric-soluble coating layer may further include plasticizers and may further include colorants, antioxidants, talc, titanium dioxide, flavoring agents, and the like. As the plasticizer, at least one component selected from the group consisting of castor oil, fatty acids, substituted triglycerides and glycerides, polyethylene glycols having a molecular weight in a range of 300 to 50,000, and derivatives thereof may be used. As the solvent of the coating solution for preparing the enteric-soluble coating layer, water or an organic solvent may be used, and as the organic solvent, ethanol, isopropanol, acetone, chloroform, dichloromethane, or a mixture thereof is preferably used.

### Advantageous Effects

As described above, the ion-binding complex containing teriparatide, according to the present disclosure, can maintain a high dose of the drug by inhibiting decomposition by peptidase in the gastrointestinal tract by incorporating the teriparatide into the micelle structure. Since the ion-binding complex can be used as various membrane passage and absorption pathways to improve drug permeability, the ion-binding complex can eventually be helpful in preventing or treating osteoporosis by increasing the oral bioavailability of the drug.

### Description of Drawings

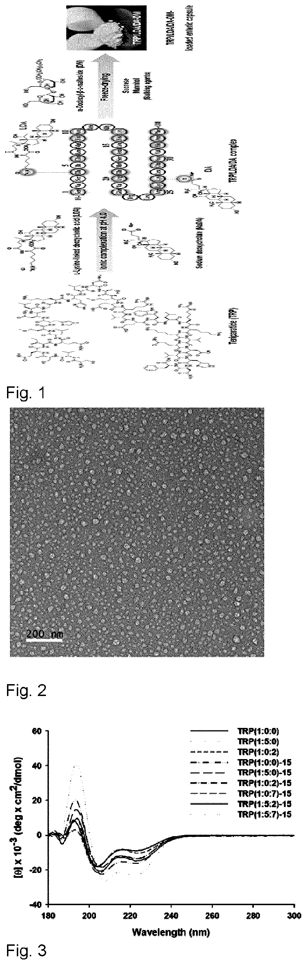

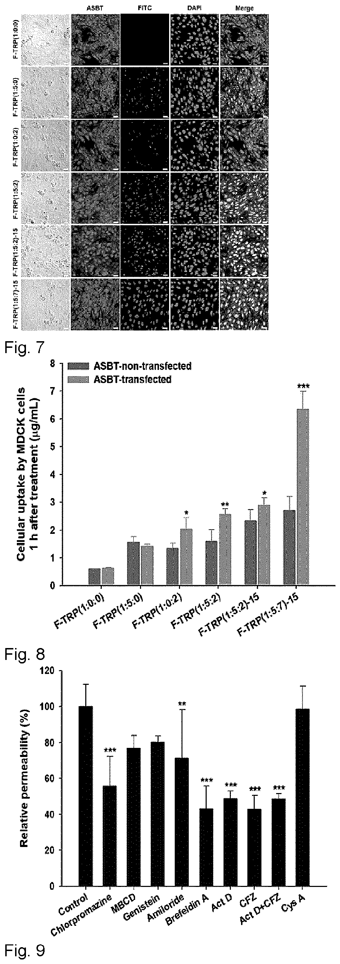

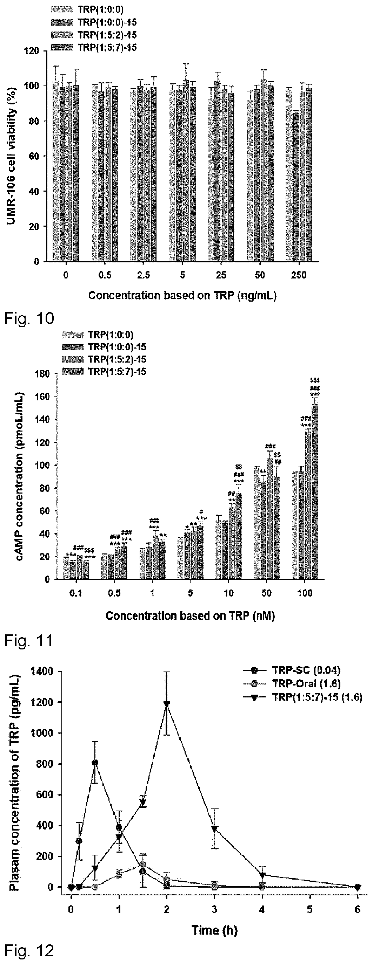

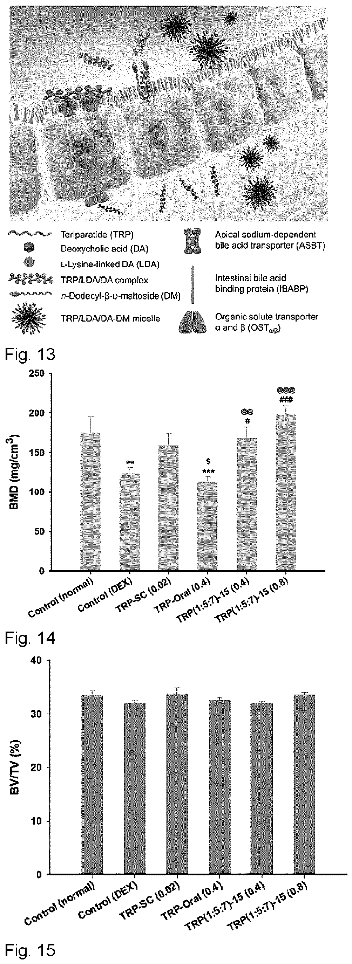

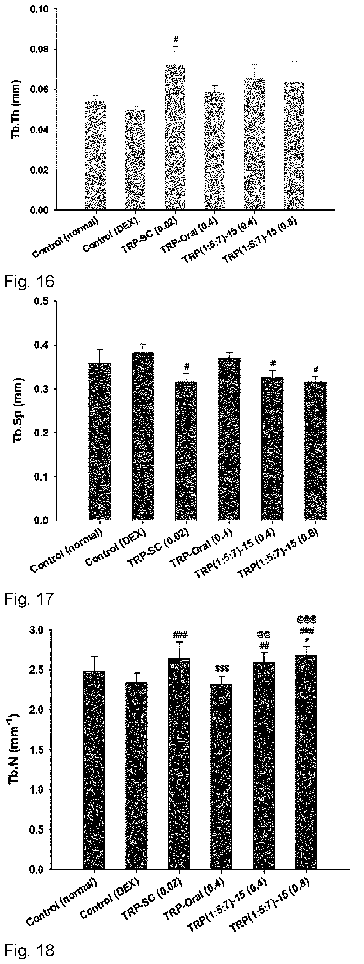

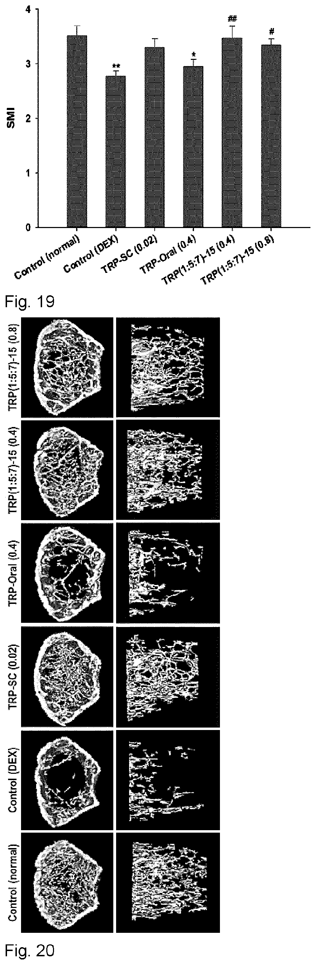

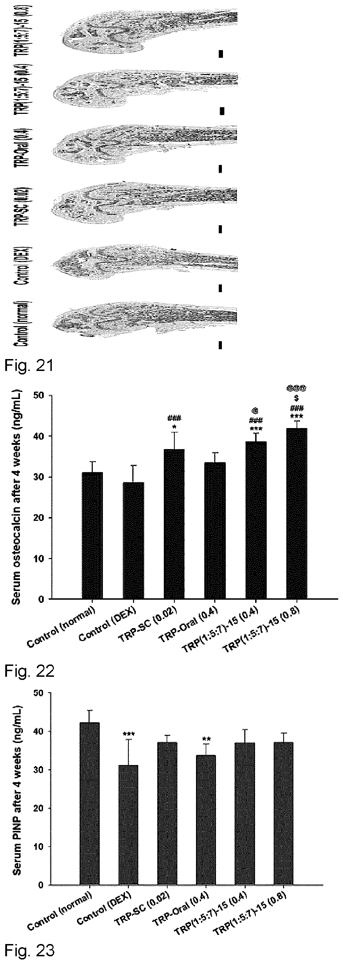

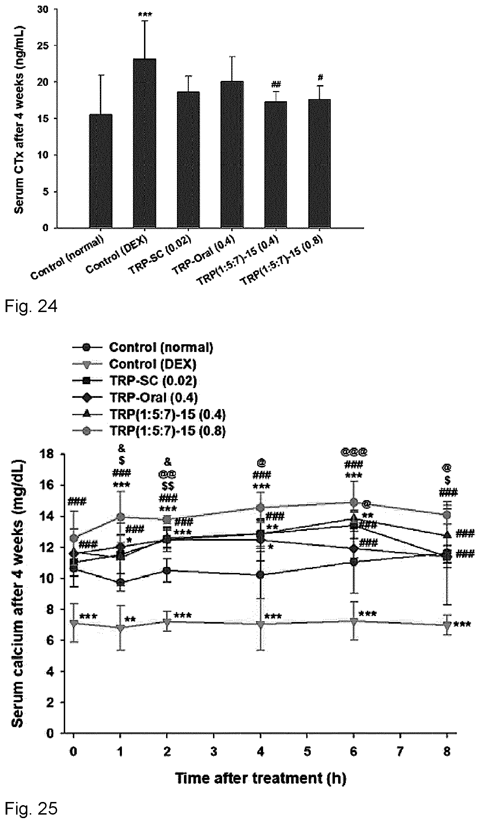

FIG. 1 is a schematic diagram showing the preparation of a solid oral formulation of teriparatide (TRP) electrostatically complexed with deoxycholic acid (DA) and l-lysine-linked DA (LDA) (TRP/LDA/DA) in the presence of n-dodecyl-β-d-maltoside (DM), an alkyl glycoside-based surfactant;
FIG. 2 is a transmission electron microscope image of TRP (1:5:7)-15;
FIG. 3 is a result of circular dichroism (CD) spectrum analysis of TRP/LDA/DA-DM composed of TRP and various complex ratios (TRP:LDA:DA) in which the TRP concentration in the aqueous phase is 10 µM;

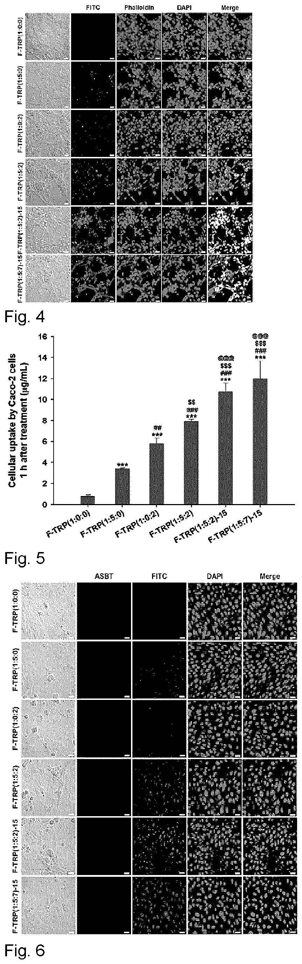

FIG. 4 is a confocal laser scanning microscope image showing cell absorption of F-TRP (1:0:0), F-TRP (1:5:0), F-TRP (1:0:2)-15, and F-TRP (1:5:7)-15 in Caco-2 cells (scale bar, 20 µm);
FIG. 5 shows Caco-2 cells F-TRP (1:0:0), F-TRP (1:5:0), F-TRP (1:0:2), F-TRP (1: 5:2), F-TRP (1:5:2), and F-TRP (1:5:7)-15, and then measuring the cellular uptake of F-TRP for 1 hour; Each value represents the mean ± SD (n = 4). ****p* < 0.001 compared to F-TRP (1:0:0); ^{##}*p* < 0.01, ^{###}*p* < 0.001 compared to F-TRP (1:5:0); ^{$$}*p* < 0.01, ^{$$$}*p* < 0.001 compared to F-TRP (1:0:2); ^{@@@}*p* < 0.001 compared to F-TRP(1:5:2).
FIGS. 6 and 7 are confocal laser scanning microscopy images of cellular uptake of F-TRP (1:0:0), F-TRP (1:5:0), F-TRP (1:0:2), F-TRP (1:5:2)-15, and F-TRP (1:5:7)-15 in non-transfected MDCK cells (FIG. 6) and ASBT-transfected MDCK cells (FIG. 7) (scale bar, 20 pm);
FIG. 8 shows the results of cellular uptake of F-TRP by ASBT-transfected/nom-transfected MDCK cells after treatment with F-TRP (1:0:0), F-TRP (1:5:0), F-TRP (1:0:2), F-TRP (1:5:2)-15, and F-TRP (1:5:7)-15 for 1 hour. Each value represents the mean ± SD (n = 4) . **p* < 0.05, ***p* < 0.01, ****p* < 0.001 compared to the uptake in ASBT-non-transfected MDCK cells treated with the same material.
FIG. 9 shows the relative apparent permeability (*P*ₐₚₚ) values of TRP (1:5:7)-15 across Caco-2/HT29-MTX-E12 cell monolayers after incubation with various transport inhibitors of the transcytosis mechanism; each value represents the mean ± SD (n = 4) . ***p* < 0.01, ****p* < 0.001, compared to the *P*ₐₚₚ of TRP (1:5:7)-15 in the absence of all inhibitors (untreated control).
FIG. 10 shows the cytotoxicity of free teriparatide [TRP (1:0:0)]; TRP on DM [TRP (1:0:0)-15]; and complexes of TRP (1:0:0)-15 with DA and LDA [TRP (1:5:2)-15 and (1:5:7)-15] after incubation with UMR-106 cells; each value represents the mean ± SD (n = 6). **p* < 0.05, ***p* < 0.01, ****p* < 0.001 compared to TRP (1:0:0) at a concentration equivalent to that of TRP; ^{#}*p* < 0.05, ^{##}*p* < 0.01, ^{###}*p* < 0.001 compared to TRP (1:0:0)-15 at a concentration equivalent to that of TRP; ^{$$}*p* < 0.01, ^{$$$}*p* < 0.001 compared to TRP (1:5:2)-15 at a concentration equivalent to that of TRP.
FIG. 11 shows the release of cAMP after incubation of free teriparatide [TRP (1:0:0)]; TRP on DM [TRP (1:0:0)-15]; and complexes of TRP (1:0:0)-15 with DA and LDA [TRP (1:5:2)-15 and (1:5:7)-15] with UMR-106 cells; each value represents the mean ± SD (n = 6). **p* < 0.05, ***p* < 0.01, ****p* < 0.001 compared to TRP (1:0:0) at a concentration equivalent to that of TRP; ^{#}*p* < 0.05, ^{##}*p* < 0.01, ^{###}*p* < 0.001 compared to TRP (1:0:0)-15 at a concentration equivalent to that of TRP; ^{$$}*p* < 0.01, ^{$$$}*p* < 0.001 compared to TRP (1:5:2)-15 at a concentration equivalent to that of TRP.
FIG. 12 is the mean plasma concentration-time profile of teriparatide after administration of a single subcutaneous injection (SC) of 0.04 mg/kg TRP (TRP-SC) that is a TRP corresponding to 1.6 mg/kg TRP [TRP-Oral (1.6)], or 1.6 mg/kg, a TRP (1:5:7)-15[TRP (1:5:7)-15 (1.6)], to mice; each value represents the mean ± SD (n = 5 for each group).
FIG. 13 is a schematic diagram of predicted specific intracellular signaling via ASBT-facilitated transport and nonspecific transcytosis of TRP (1:5:7)-15 through the intestinal epithelium;
FIGS. 14 to 19 show the results of measuring various parameters through micro-CT of femurs harvested from the mice of normal untreated mice [control group (normal)], dexamethasone (DEX)-induced osteoporotic mice [control group (DEX)], and mice that have been co-treated with a once daily subcutaneous (SC) infusion of 0.02 mg/kg teriparatide (TRP; TRP-SC (0.02)) for 4 weeks, or orally administrated of 0.4 mg/kg TRP [TRP-oral (0. 4)], 0.4 mg/kg TRP [TRP (1:5:7)-15 (0.4)] as TRP (1:5:7)-15, or TRP(1:5:7)-15, 0.8 mg/kg TRP [TRP(1:5:7)-15 (0.8)] once daily for 4 weeks. Bone mineral density (BMD, Fig. 14), bone volume fraction (BV/TV, FIG. 15), bone thickness (Tb.Th, FIG. 16), bone separation (Tb.Sp, FIG. 17), bone number (Tb.N, FIG. 18) and structural model index (SMI, FIG. 19). **p* < 0.05, ***p* < 0.01, ****p* < 0.001 compared to the control (normal); ^{#}*p* < 0.05, ^{##}*p* < 0.01, ^{###}*p* < 0.001 compared to the control (DEX); ^{$}*p* < 0.05, ^{$$$}*p* < 0.001 compared to TRP-SC (0.02); ^{@@}*p* < 0.01, ^{@@@}*p* < 0.001 compared to TRP-Oral (0.4).
FIG. 20 is a micro-computed tomography (micro-CT) image of a control group (normal) or dexamethasone (DEX) induced osteoporotic mice [control group (DEX)] or mice co-treated with TRP-SC (0.02), TRP-oral (0.4), TRP (1:5:7)-15 (0.4), or TRP (1:5:7)-15 (0.8) for 4 weeks;
FIG. 21 is a longitudinal H&E-stained histologic image of a distal femora from a control group (normal) or dexamethasone (DEX)-induced osteoporotic mice [control group (DEX)] or mice co-treated with TRP-SC (0.02), TRP-oral (0.4), TRP (1:5:7)-15 (0.4), or TRP (1:5:7)-15 (0.8) for 4 weeks; and
FIGS. 22 to 25 are the results of analyzing the bone formation and serum absorption markers from untreated mice [control group (normal)] or mice treated with dexamethasone (DEX) [control group (DEX)], or mice treated with 0.4 mg/kg TRP [TRP (1:5:7)-15(0.4)] or TRP (1:5:7)-15 0.8 mg/kg TRP [TRP (1:5:7)-15(0.8)] as 0.02 mg/kg TRP [TRP; TRP-SC (0. 02)] or 0.4 mg/kg TRP [TRP-Oral (0.4)], TRP (1:5:7)-15. (FIG. 22, osteocalcin; FIG. 23, procollagen I N-terminal pro-peptide (PINP); FIG. 24, C-terminal cross-linked telopeptide of type I collagen (CTx); and FIG. 25, calcium level at day 28 after treatment).

### Mode for Invention

Hereinafter, the present disclosure will be described in more detail through Examples. Since these examples are intended to illustrate the present disclosure only, the scope of the present disclosure is not to be construed as being limited by these examples.

### Preparation Example 1: Material

Human PTH (1-34) acetate (TRP) was purchased from PolyPeptide Laboratories, Inc. (Torrance, CA, USA). Deoxycholic acid (DA), sodium deoxycholate (NaDA), n-dodecyl-β-D-maltoside (DM), sucrose, mannitol, chlorpromazine, methyl-β-cyclodextrin (MBCD), brefeldin A, genistein, actinomycin D (Act D), cyclosporine A (Cys A) and clofazimine (CFZ) were purchased from Sigma-Aldrich (St. Louis, MO, USA). Solvents for HPLC and UPLC-MS/MS analysis were purchased from Merck Group (Darmstadt, Germany) and Thermo Fisher Scientific (Waltham, MA, USA).

### Preparation Example 2: Experimental animals

Sprague-Dawley (SD) mice (male, 6-7 weeks old, 200-250 g) and C57LB/6 mice (female, 8-9 weeks old, 20-25 g) were purchased from G-bio (Gwangju, Korea). Ethical approval was obtained from the Animal Care and Use Committee of Mokpo National University (Jeonnam, Republic of Korea, approval numbers MNU-IACUC-2021-006 and MNU-IACUC-2021-018). All animal experiments were performed in accordance with the Guidelines for the Care and Use of Laboratory Animals of the National Institutes of Health and the Institutional Animal Care and Use Committee guidelines.

### Examples and Comparative Examples: Preparation of TRP/LDA/DA-DM ion-binding complexes

LDA, an oral penetration enhancer, was synthesized by binding positively charged L-lysine with DA. Next, TRP was dissolved at a concentration of 1 mg/mL in water and adjusted to pH 4.0 by the addition of 1% acetic acid. LDA and NaDA were dissolved in water (adjusted to pH 4.0 with 1% acetic acid) at concentrations of 2.78 mg/mL and 2.82 mg/mL, respectively. Then, 1 mL of TRP solution (1 mg/mL) was electrostatically complexed with LDA in molar ratios of 1:1, 1:2, 1:3, 1:4, and 1:5 (TRP/LDA), and NaDA solution was added to form electrostatic complexes between TRP and DA in molar ratios of 1:1, 1:2, 1:5, 1:7, and 1:14, respectively. As a result of the formation of the complex, TRP aggregated and precipitated at a TRP/DA molar ratio > 1:2. Accordingly, DM, an alkyl glycoside-based surfactant, was introduced into the TRP solution prior to complex formation so that the weight ratio of TRP:DM was 1:1 to 1:15. The ion-binding complexes (TRP/LDA/DA-DM) prepared in the absence and presence of DM were added to the formulation with 1 mL of sodium acetate buffer solution (pH 4.0) containing 25 mg of mannitol and 5 mg of sucrose, and the final mixture was freeze-dried to obtain a solid powder. Specific mixing ratios and yields are shown in Table 1.

**[Table 1]**

| | **Compositi on ratio** | **TRP (mg )** | **LDA (mg)** | **DA (mg)** | **DM (mg )** | **Mannit ol (mg)** | **Sucro se (mg)** | **Tota l (mg)** |
|---|---|---|---|---|---|---|---|---|
| Example 1 | TRP(1: 5: 2) -15 | 1 | 0.69 0 | 0.19 6 | 15 | 25 | 5 | 46.8 86 |
| Example 2 | TRP(1: 5: 7) -15 | 1 | 0.69 0 | 0.68 6 | 15 | 25 | 5 | 47.3 76 |
| Example 3 | TRP(1:5:1 4)-15 | 1 | 0.69 0 | 1.37 2 | 15 | 25 | 5 | 48.0 62 |
| Comparat ive Example 1 | TRP(1: 0: 0) | 1 | | | | 25 | 5 | 31.0 |
| Comparat ive Example 2 | TRP(1: 5: 0) | 1 | 0.69 0 | | | 25 | 5 | 31.6 9 |
| Comparat ive Example 3 | TRP(1: 0: 1) | 1 | | 0.09 8 | | 25 | 5 | 31.0 98 |
| Comparat ive Example 4 | TRP(1: 0: 2) | 1 | | 0.19 6 | | 25 | 5 | 31.1 96 |
| Comparat ive Example 5 | TRP(1: 5: 2) | 1 | 0.69 0 | 0.19 6 | | 25 | 5 | 31.8 86 |
| Comparat ive Example 6 | TRP(1: 0: 0) -5 | 1 | | | 5 | 25 | 5 | 36.0 |
| Comparat ive Example 7 | TRP(1: 0: 0) -15 | 1 | | | 15 | 25 | 5 | 36.0 |
| Comparat ive Example 8 | TRP(1: 5: 0) -15 | 1 | 0.69 0 | | 15 | 25 | 5 | 46.0 69 |
| Comparat ive Example 9 | TRP(1: 0: 2) -15 | 1 | | 0.19 6 | 15 | 25 | 5 | 46.1 96 |
| Comparat ive Example 10 | TRP(1: 0: 7) -15 | 1 | | 0.68 6 | 15 | 25 | 5 | 46.6 86 |
| Comparat ive Example 11 | TRP(1: 0: 14) -15 | 1 | | 1.37 2 | 15 | 25 | 5 | 47.3 72 |

In the process of preparing ion-binding complexes according to the above compositions and ratios, the optimal formulation ratio was derived. In detail, during the above ion-binding complex preparation, TRP formed ion-binding complexes with LDA in a molar ratio of 1:1 to 1:5, and no signs of aggregation or precipitation were observed up to TRP/LDA (1:5) due to the positive charge remaining on the TRP molecule, and the drug content after drying was the same as that of natural TRP. Furthermore, at TRP: DA molar ratios > 1:2, TRP precipitated out of solution, resulting in 13.6%, 22.4%, 40.3%, 59.1%, and 94.1% lower drug content than TRP (1:0:3), (1:0:4), (1:0:6), (1:0:10), and (1:0:14), respectively. This can cause stability problems, impair bioactivity, and reduce the permeability and bioavailability of TRP, it was estimated that the maximum ion-binding complexation molar ratio of LDA and DA to TRP (TRP/LDA/DA) that can complex with LDA and DA without precipitation to be 1:5:2. The inventors then introduced DM into the TRP solution as a dispersant that could protect the protein or peptide from aggregation without causing oxidative degradation, and found that TRP conjugated maximally with DA up to TRP(1:5:14)-15 without aggregation or loss of TRP. Furthermore, in all TRP/LDA/DA complexes (i.e., with and without DM), free DA and LDA were not detected in the ultrafiltrates before and after lyophilization, confirming that LDA and DA formed complexes with TRP at all molar ratios.

### Experimental Example 1: Confirmation of characteristics of RP/LDA/DA-DM

To confirm the formation of an ion-binding complex, TRP/LDA, TRP/DA, or TRP/LDA/DA was diluted to a concentration of 10 to 1,000 ug/mL with water before and after TRP lyophilization. Each ion-binding complex solution was then loaded into an ultrafiltration spin column (Pierce Protein Concentrator, molecular weight cut-off [MWCO] = 3,000; Thermo Fisher Scientific) and centrifuged at 12,000 × g for 30 min to separate non-complexed DA or LDA from the solution. The free DA in the filtrate was then detected at 210 nm using an HPLC equipped with a Luna C18 column (4.6 × 150 mm, 5 µm, 100 Å) at 35°C. The mobile phase was acetonitrile, 20 mM sodium acetate buffer (pH 4.3, adjusted with acetic acid), and methanol mixture (60:35:5, v/v/v), and the analytical conditions were flow rate 1.5 mL/min. In the filtrate, LDA was performed at a temperature of 40°C and a detector wavelength of 210 nm, and the column was Luna C18 (4.6 × 250 mm, 5 µm, 100 Å). The mobile phase consisted of a pH 3.0 buffer consisting of 0.7% (v/v) trimethylamine and 0.1% hexane sulfonic acid sodium salt, and the flow rate was 0.8 mL/min.

The characterization of the secondary structure of RP/LDA/DA-DM was confirmed by far-field dichroism spectroscopy of TRP, TRP-DM, TRP/LDA/DA, and TRP/LDA/DA-DM, using 10 µM of the complex in water at 20°C. Measurements were made in the 180 to 260 nm range on a JASCO spectropolarimeter (Jasco Inc., Easton, MD, USA) using a 10 mm cuvette. All spectra were background-corrected and calculated in units of average residual ellipticity. This represents an average of 10 scan values. The optimized TRP/LDA/DA-DM [i.e., TRP(1:5:7)-15] was also characterized for average particle size, polydispersity index (PDI), and zeta potential using a dynamic light scattering analyzer (Malvern Zetasizer Nano ZS90; Malvern Instruments, Malvern, UK) at 25°C. TRP/LDA/DA-DM was dispersed in deionized water and sonicated for 1 min to minimize multi-scattering effects. In addition, morphological evaluation of the TRP formulation was performed by transmission electron microscopy (TEM). The diluted dispersion was placed on a copper grid, excess fluid was removed using filter paper, and negative staining was performed with a 2% aqueous solution of phosphortungstic acid. The prepared grids were observed by high-resolution TEM (HRTEM; JEM-200; JEOL, Tokyo, Japan). The results are shown in Table 2 and FIGS. 2 and 3.

As a result, the optimal formulation [i.e., TRP (1:5:7)-15] has a particle size of 7.64 ± 0.098 nm, a PDI of 0.160 ± 0.034, and a zeta potential of -0.361 ± 0.154 mV (Table 2). Furthermore, the morphology and structure of the oral formulation determined by TEM were found to be in the form of uniform nano-sized micelles with a diameter of less than 10 nm based on particle size analysis (FIG. 2).

Furthermore, the secondary structure of TRP/LDA/DA-DM confirmed that the complexes of native TRP with LDA and/or DA in the presence or absence of DM have the same negative intensity at 222 nm and exhibit an α-helical structure. Furthermore, the electrostatic complexation of TRP with LDA and DA by DM did not affect the secondary structure of natural TRP (FIG. 3).

**[Table 2]**

| | **Particle size (nm)** | **Polydispersity index (PDI)** | **Zeta potential (mV)** |
|---|---|---|---|
| DM | 7.43 ± 0.081 | 0.045±0.017 | -0.260 ± 0.340 |
| TRP(1: 5: 0) -15 (Comparative Example 8) | 5.03 ± 0.262 | 0.222 ± 0.084 | -0.261 ± 0.341 |
| TRP(1: 0: 7) -15 (Comparative Example 10) | 8.71 ± 0.173 | 0.112 ± 0.015 | -0.375 ± 0.015 |
| TRP(1: 5: 2) -15 (Example 1) | 5.44 ± 0.552 | 0.188 ± 0.056 | -0.104 ± 0.170 |
| TRP(1: 5: 7) -15 (Example 2) | 7.64 ± 0.098 | 0.160 ± 0.034 | -0.361 ± 0.154 |

### Experimental Example 2: Confirmation of in vitro intestinal permeability of TRP/LDA/DA-DM

### 2-1. Artificial intestinal permeability in vitro

Improved TRP permeation of TRP/LDA/DA or TRP/LDA/DA-DM through the lipid bilayer was confirmed through parallel artificial membrane (PAMPA) analysis (BD Biosciences, San Jose, CA, USA). Briefly, 300 µL of phosphate-buffered saline (PBS, pH 6.8) was added to each receiver plate well, and 200 µL of TRP, or an electrostatic complex of TRP with LDA and/or DA in the absence or presence of DM in PBS (pH 6.8), based on 100 ug/mL of TRP, was added to each well of the filter plate. After combining the plates, they were incubated for 5 hours at room temperature without shaking. The TRP concentration (µg/mL) permeated through the artificial membrane was quantified by HPLC at 210 nm using an Agilent Poroshell 120 SB-C8 (4.6 × 150 mm, 2.7 µm) analytical column as the stationary phase. An aliquot (30 µL) of each sample was eluted for 32 min in 0.1 M sodium perchlorate (pH 2.7) mobile phase with a flow rate of 1.0 mL/min and a linear gradient of 29%-45% acetonitrile. The results are shown in Table 3 below.

**[Table 3]**

| | Composition ratio | Permeated concentration of TRP across an artificial membrane (µg/mL) | Apparent permeability (*P*ₐₚₚ, X 10⁻⁶ cm/s) |
|---|---|---|---|
| Example 1 | TRP(1: 5: 2) -15 | 46.1 ± 5.10 | 0.690 ± 0.086 |
| Example 2 | TRP(1: 5: 7) -15 | 49.1 ± 2.30 | 0.750 ± 0.091 |
| Example 3 | TRP(1:5:14)-15 | 44.8 ± 3.52 | 0.730 ± 0.012 |
| Comparative Example 1 | TRP(1: 0: 0) | N.D. | 0.181 ± 0.033 |
| Comparative Example 2 | TRP(1: 5: 0) | 3.679 ± 0.108 | 0.191 ± 0.022 |
| Comparative Example 3 | TRP(1: 0: 1) | N.D. | 0.180 ± 0.041 |
| Comparative Example 4 | TRP(1: 0: 2) | 4.70 ± 0.933 | 0.185 ± 0.026 |
| Comparative Example 5 | TRP(1: 5: 2) | 11.8 ± 4.48 | 0.212 ± 0.030 |
| Comparative Example 6 | TRP(1: 0: 0) -5 | 56.1 ± 4.60 | 0.379 ± 0.010 |
| Comparative Example 7 | TRP(1: 0: 0) -15 | 48.7 ± 1.60 | 0.463 ± 0.091 |
| Comparative Example 8 | TRP(1: 5: 0) -15 | 50.9 ± 2.95 | 0.493 ± 0.071 |
| Comparative Example 9 | TRP(1: 0: 2) -15 | 43.6 ± 5.20 | 0.573 ± 0.010 |
| Comparative Example 10 | TRP(1: 0: 7) -15 | 53.7 ± 6.70 | 0.620 ± 0.080 |
| Comparative Example 11 | TRP(1: 0: 14) -15 | 54.7 ± 3.50 | 0.620 ± 0.060 |

Permeate concentration (µg/mL) and apparent permeability (Papp) of TRP with or without LDA and/or DA alone or in combination with DM. Each value represents the mean ± SD (n = 6). TRP, teriparatide; DA, deoxycholic acid; LDA, L-lysine-linked DA; DM, n-dodecyl-β-D-maltoside; TRP/LDA, TRP complexed with LDA; TRP/DA, TRP complexed with DA; TRP/LDA/DA-DM, TRP complexed with LDA and DA in the presence of DM; N.D., not detected.

As shown in Table 3 above, TRP (1:0:0) or TRP (1:0:1) could not be detected in the receptor wells of the PAMPA plate after 5 hours of permeation, whereas TRP penetrated the artificial membrane after complex formation with LDA or DA at a molar ratio of 1:5 or 1:2 [i.e., TRP (1:5:0) and TRP (1:0:2)]. Furthermore, TRP (1:5:2) showed a 321% and 251% increase in permeation compared to TRP (1:5:0) and TRP (1:0:2), respectively, indicating a significant improvement in passive diffusion through the artificial membrane. Furthermore, the increased lipophilicity of TRP in DM micelles was confirmed by the fact that TRP incorporated into DM at 1:5 and 1:15 weight ratios [i.e., TRP (1:0:0)-5 and TRP (1:0:0)-15] exhibited 4.75 times and 4.12 times greater permeation than TRP (1:5:2), respectively. However, there was no significant difference in TRP permeation between TRP (1:0:0)-5 and TRP (1:0:0)-15, and RP (1:0:0)-15 was selected for maximum complexation of LDA and DA with TRP in order to maintain intact TRP-micelle interactions, considering the dose or rapid dilution that may occur in GI fluids. In addition, TRP (1:5:0)-15, TRP (1:0:2)-15, TRP (1:0:7)-15, and TRP (1:0:14)-15 with DM incorporated with TRP/LDA/DA showed TRP-like permeation compared to TRP (1:0:0)-15. These results show that at pH 6.8, as opposed to pH 4.0, the polarity of TRP decreased significantly, which increased the lipid solubility of TRP, resulting in maximum permeation through the artificial membrane with DM micelles.

### 2-2. In vitro permeability through Caco-2 and HT-29-MTX-E12 cell monolayers

To evaluate the enhanced intestinal epithelial uptake of TRP, TRP/DLA/DA, and TRP/DLA/DA-DM, cells mixed 8:2 with Caco-2 (ATCC HTB-37^{™}; American Type Culture Collection, Manassas, VA, USA) and HT29-MTX-E12 (EACC 12040401; Public Health, Oxford, England) were inoculated into 24-well Transwell^{®} filter membranes at a density of 1 × 10⁵ cells/well. After obtaining a confluent monolayer with transepithelial electrical resistance (TEER) > 650 ± 100 Ω.cm², the apical and basal compartments of the Transwell^{®} system were washed with pre-warmed Hank's balanced salt solution (HBSS) and maintained at 37°C for 30 min. For permeation experiments, the HBSS in the apical chamber was replaced with 0.1 mL of TRP, TRP/LDA, TRP/DA, TRP-DM, TRP/LDA-DM, TRP/DA-DM, or TRP/LDA/DA-DM diluted in HBSS to correspond to 200 µg/mL TRP. Each basolateral compartment was replaced with 0.6 mL of fresh HBSS, and the Transwell^{®} plate was incubated at 37°C ± 0.5°C. After 0.5, 1, 2, 3, 0.5, 1, 2, 3, 4, and 5 hours, 0.2 mL of each sample solution was collected from the basolateral compartment and replaced with the same volume of HBSS. TEER values for monolayers were measured again at the end of the experiment. The collected samples were then filtered through a 0.45 um polyvinylidene difluoride (PVDF) membrane, and the permeation of TRP was determined by HPLC using a UV detector at 210 nm as described above. The apparent permeability (Papp) of TRP in the formulation was calculated by the following equation. Papp = dM/dt × 1/(A × Co), where dM/dt represents the slope of the permeated TRP accumulation against time (µg/h), A is the surface area of the monolayer (cm²), and Co is the initial loading concentration of TRP at the apical side (pg/ML). The results are shown in Table 3 below.

As shown in Table 3, unlike the artificial membrane permeation, TRP (1:5:0), TRP (1:0:1), and TRP (1:0:2) had almost the same permeability when compared to free TRP(1:0:0). Furthermore, TRP (1:5:2) showed only a 117% increase in *P*ₐₚₚ over TRP, indicating that there was no effect of ASBT-mediated transport and that the permeation of TRP could be mainly due to simple passive diffusion. Furthermore, despite the increased relative hydrophobicity of TRP (1:5:2) compared to free TRP, it did not show strong interaction with several transmembrane regions of ASBT. Therefore, to further increase the interaction with ASBT, we combined TRP (pH4) with a micellar solution of DM for maximum complexation of DA with TRP and found that TRP (1:0:0)-5 and -15 increased the permeability of TRP by 2.09 times and 2.56 times, respectively, compared to TRP (1:0:0), and the permeability of TRP(1:0:0)-15 was 218% greater than TRP(1:5:2). In addition, the TEER value decreased by 4.33 times compared to the TRP-treated monolayer after permeation. Furthermore, TRP (1:5:7)-15 exhibits 162% and 414% greater Papp compared to TRP (1:0:0)-15 and TRP (1:0:0), respectively, suggesting that ASBT-mediated transport for TRP is also involved in the additional permeability increase of TRP (1:5:7)-15.

### Experimental Example 3: Confirmation of cellular uptake of FITC-conjugated TRP/LDA/DA-DM by Caco-2 and ASBT transfected MDCK cells

To confirm the enhanced cellular uptake of TRP/DLA/DA and TRP/DLA/DA-DM, TRP was conjugated with fluorescein isothiocyanate (FITC) at a molar ratio of 1:1 using the FluoroTag^{™} FITC Conjugation Kit (Sigma-Aldrich). Briefly, 0.54 mL of FITC dissolved in 0.1 M sodium carbonate-bicarbonate buffer (1 mg/mL) was added to 0.5 mL of TRP solution (12 mg/mL) in 0.1 M sodium carbonate-bicarbonate buffer (pH 9.0) and incubated for 2 hours at room temperature with gentle agitation. The FITC-labeled TRP (F-TRP) was then eluted with a reaction solution containing 10 mL of PBS (pH 9.0) and separated through a Sephadex G-25M column. The collected F-TRP was lyophilized and subjected to further electrostatic complexation with LDA and/or DA in the presence or absence of DM to form F-TRP/LDA/DA or F-TRP/LDA/DA-DM as described above.

Next, the cellular uptake of F-TRP, F-TRP/LDA/DA or F-TRP/LDA/DA-DM was analyzed qualitatively in Caco-2 cells. Briefly, Caco-2 cells were inoculated on Cell-Tak-coated coverslips at 5 × 10⁴ cells/coverslip and grown until a monolayer was formed. The cells were incubated at 37°C after replacement with culture medium containing 100 µL of F-TRP (1:0:0), F-TRP (1:5:0), F-TRP (1:0:2), F-TRP (1:5:2), F-TRP (1:5:2)-15, and F-TRP (1:5:7)-15 solutions dissolved in DMEM corresponding to a TRP concentration of 20 µg/mL. After 1 hour, the cells were washed with ice-cold PBS (pH 7.4) and fixed with PBS (pH 7.4) containing 4% formaldehyde. The actin filaments of the cells were then stained with phalloidin-rhodamine (100 nM), and the nuclei were counterstained with 4',6-diamidine-2'-phenylindole dihydrochloride (DAPI). After lysing with PBS (pH 7.4) three times, cell uptake was confirmed by confocal laser scanning microscopy (Carl Zeiss, Oberkochen, Germany), and the results are shown in FIGS. 4 and 5.

As can be seen in FIG. 4, F-TRP (1:5:2)-15 showed significantly higher uptake than F-TRP (1:0:0) and F-TRP (1:5:2). It can also be seen that after ion-binding complexation with LDA and DA, the permeability of TRP (1:5:2)-15 and TRP (1:5:7)-15 was significantly enhanced compared to F-TRP (1:0:0) and F-TRP (1:5:2). Furthermore, as shown in Figure 5, after exposing Caco-2 cells to F-TRP (1:5:0) and F-TRP (1:0:2) for 1 hour, the cellular uptake of F-TRP (1:5:0) and F-TRP (1:0:2) was 3.38 ± 0.111 µg/mL and 5.81 ± 0.537 µg/mL, respectively, which were 4.28 and 7.35 times higher than the negative F-TRP (1:0:0), respectively. Cellular uptake of F-TRP in F-TRP (1:5:2) increased by 133% and 35.8% compared to F-TRP (1:5:0) and F-TRP (1:0:2), respectively, and was 9.99 times higher than F-TRP (1:0:0). Furthermore, exposure of Caco-2 cells to F-TRP (1:5:7)-15 for 1 hour further increased cellular uptake to 12.0±1.70 µg/mL, which was 1.52 and 15.2 times higher than F-TRP (1:5:2) and F-TRP (1:0:0), respectively.

Next, the enhancement of intestinal permeability of TRP/LDA/DA or TRP/LDA/DA-DM through interaction with bile acid transporters after complexation of TRP with LDA and/or DA was determined using Mardin-Darby canine kidney (MDCK) cells (ATCC CCL-34^{™}) transfected with the human ASBT gene. Briefly, MDCK cells were inoculated at a density of 1 × 10⁴ cells/coverslip and then transfected with the human ASBT gene using Lipofectamine 2000 and P300^{™} (Thermo Fisher Scientific). After the formation of dense monolayers, cells were treated with 100 µL of F-TRP, F-TRP/LDA/DA, or F-TRP/LDA/DA-DM diluted in DMEM to a concentration equivalent to 20 µg/mL TRP and incubated at 37°C for 1 hour. After washing three times with ice-cold PBS (pH 7.4), cells were fixed with 4% formaldehyde and blocking buffer (0.3% Triton X-100 and 10% normal goat serum in PBS, pH 7.4). After treatment with anti-human ASBT antibody (1:500) and incubation overnight, the cells were stained with 10 µg/mL Alexa Fluor-546 labeled secondary antibody for 1 hour. Finally, nuclei were counterstained with DAPI for 5 minutes, and cells were fixed to glass slides using mounting solution. Fluorescent images were obtained with a confocal laser scanning microscope. The results are shown in FIGS. 6 to 7.

As can be seen in FIGS. 5 and 6, confocal images of F-TRP (1:5:2 and 1:5:7)-15 showed higher cellular uptake in ASBT-expressing MDCK cells than in non-expressing cells. In particular, F-TRP (1:5:7)-15 showed higher cellular uptake than F-TRP (1:5:2)-15 in ASBT-expressing MDCK cells.

Finally, Caco-2 and ASBT-non-transfected or transfected MDCK cells were treated with F-TRP (1:5:0), F-TRP (1:0:2), F-TRP (1: 5:2), F-TRP (1:5:2)-15, and F-TRP (1:5:7)-15 were quantified. Briefly, 1 × 10⁵ Caco-2 or MDCK cells were inoculated into each well of a 6-well plate containing DMEM with 10% FBS and 1% penicillin/streptomycin. In parallel, MDCK cells were transfected with the ASBT gene as described above. After forming a dense monolayer, the cells were treated with each F-TRP formulation at a concentration corresponding to 50 µg/mL TRP and then incubated at 37°C for 1 hour. Then, the cells in each well were washed three times with 1 mL ice-cold PBS (pH 7.4) and harvested using a cell scraper. After centrifugation at 5000 rpm for 5 minutes, the pellet was resuspended and washed with ice-cold PBS (pH 7.4). Then, the collected cells were treated with 0.5% (w/v) Triton-X 100 containing 0.3 mL of HCl, followed by sonication for 15 minutes to disrupt cell membranes. After centrifugation, F-TRP in the supernatant was quantified by HPLC using a fluorescence detector at an excitation wavelength of 495 nm and an emission wavelength of 525 nm. The results are shown in Table 8 below.

As can be seen in FIG. 8, TRP complexed with LDA and/or DA increased cellular uptake of TRP into ASBT-non-expressing cells. Although F-TRP (1:5:2) showed 269% higher uptake than F-TRP (1:0:0), the uptake of TRP/LDA complexes by ASBT-non-expressing MDCK cells was similar. After adding DM to the complex, uptake of TRP (1:5:2)-15 increased by 46.5% compared to F-TRP (1:5:2), whereas that of F-TRP (1:5:7) did not increase anymore. On the other hand, uptake of the complex through ASBT-expressing cells was significantly enhanced in the complex composed of two or more DA molecules in F-TRP. After treatment of F-TRP (1:5:7)-15 to ASBT-non-expressing MDCK cells for 1 hour, the maximum F-TRP concentration was 2.71 ± 0.506 µg/mL, which was 2.71 ± 0.506 µg/mL, and F-TRP (1:0:0) and was 4.59 times and 1.16 times higher than F-TRP(1:5:2)-15 cells, respectively. Furthermore, F-TRP (1:5:7)-15 showed 234% greater uptake by ASBT-expressing MDCK cells compared to ASBT-non-expressing MDCK cells. In other words, the cellular uptake of F-TRP (1:5:7)-15 by ASBT-expressing MDCK cells was 10.1 and 2.20 times higher than that of F-TRP (1:0:0) and F-TRP (1:5:2)-15, respectively.

### Experimental Example 4: Confirmation of the intestinal transport mechanism of TRP/LDA/DA-DM

In order to clearly confirm the involvement of the intercellular pathway during the permeation of TRP/LDA/DA-DM, Caco-2/HT29-MTX-E12 cell monolayers with TEER values > 650 Ω.cm² were prepared as described in Example 2. The monolayers were preincubated in 0.1 mL of HBSS containing specific inhibitors of each cellular uptake pathway, and 0.6 mL of HBSS not containing specific inhibitors was added to the apical and/or basal chambers for 30 min at 37°C, respectively. The solution in the apical chamber was then replaced with 0.1 mL of TRP (1:5:7)-15 (200 ug/mL TRP) with the corresponding inhibitor, while the basolateral compartment solution was replaced with 0.6 mL of fresh HBSS. Cells were incubated at 37°C, and 200 µL aliquots of the sample solution were withdrawn from the basolateral compartment at 0.5, 1, 2, 3, 4, and 5 hours and replaced with an equal volume of HBSS. The TRP that penetrated through the Caco-2/HT29-MTX-E12 cell monolayer was quantified by HPLC with a UV detector at a wavelength of 10 nm as described above. Finally, *P*ₐₚₚ was determined as the linear slope of the cumulative permeation of TRP in TRP (1:5:7)-15 plotted as a function of time using the Formula described in Example 2. The obtained results are shown in FIG. 9.

First, in order to confirm the role of different intercellular uptake pathways involved in the intestinal uptake of TRP (1:5:7)-15, the transport mechanism of TRP was determined using Caco-2/HT29-MTX-E12 monolayers in the presence of various biochemical inhibitors specific for transcytosis. As shown in FIG. 9, the effect of the inhibitors on the *P*ₐₚₚ of TRP (1:5:7)-15 was quantified and compared to the control group without inhibitors, and after treatment of the monolayer with chlorpromazine, an inhibitor of clathrin-mediated endocytosis, the Papp value of TRP (1:5:7)-15 was significantly reduced by 44.4% compared to the control group without inhibitors. Similarly, treatment with MBCD and genistein, inhibitors of caveola-mediated endocytosis, reduced the membrane transport of TRP (1:5:7)-15 by 23.2% and 19.7%, respectively, compared to the control group without inhibitors. Furthermore, inhibition of macropinocytosis by amiloride reduced the *P*ₐₚₚ of TRP (1:5:7)-15 by 28.7% compared to the control group without inhibitor.

Next, to confirm the involvement of the ER/Golgi pathway during endosomal transport of TRP (1:5:7)-15, the effect of brefeldin A, a specific and potent inhibitor that can specifically inhibit the secretory ER/Golgi pathway, was determined. As a result, as can be seen in FIG. 9, it was confirmed that the *P*ₐₚₚ of TRP (1:5:7)-15 decreased by 56.9% compared to the control group without the brefeldin A inhibitor.

Furthermore, to confirm the involvement of ASBT-mediated transporters in the cellular uptake of TRP (1:5:7)-15, Act D and CFZ were treated alone or in combination with a specific inhibitor of ASBT and a specific inhibitor of heterotrimeric organic transporters (OSTα/α), and the effect on *P*ₐₚₚ of TRP (1:5:7)-15 was compared to the control group without inhibitors. As a result, as can be seen in FIG. 9, Act D alone reduced the *P*ₐₚₚ of TRP (1:5:7)-15 by 51.2% compared to the control group without an inhibitor. CFZ, an inhibitor of OSTα/α, also reduced *P*ₐₚₚ by 57.1% compared to the control group without an inhibitor. In addition, combined treatment with Act D and CFZ reduced *P*ₐₚₚ by 51.5% compared to the control group without inhibitor.

Finally, in order to confirm the involvement of P-glycoprotein (P-gp)-mediated efflux during intestinal permeation of TRP (1:5:7)-15, the effect of Cys A, a P-gp inhibitor, was investigated in the cell monolayer during apical-to-basal transport. The *P*ₐₚₚ of TRP (1:5:7)-15 was the same as that of the control group, confirming that there was no P-gp-mediated efflux during intestinal transport of TRP (1:5:7)-15.

### Experimental Example 5: Confirmation of in vitro cytotoxicity and biological activity of TRP/LDA/DA-DM

The cytotoxic effect of TRP/LDA/DA-DM was confirmed using a murine osteosarcoma cell line (UMR-106; ATCC: CRL-1661^{™}). Briefly, 4×10⁴ cells were inoculated into each well of a 96-well plate in 100 µL of DMEM supplemented with 10% (v/v) FBS and 1% penicillin/streptomycin and incubated at 37°C for 48 hours. Cells were then treated with serial dilutions of TRP, TRP-DM, and TRP/LDA-DM in low serum medium (1% FBS) to 0.5, 2.5, 5, 25, 50, and 250 ng/mL TRP. After incubation for an additional 24 hours, 10 µL of WST-8 [2-(2-methoxy-4-nitrophenyl)-3-(4-nitrophenyl)-5-(2,4-disulfophenyl)-2H-tetrazolium monosodium salt; Dojindo Molecular Technologies, Rockville, MD, USA] was added to each well and incubated for an additional 2 hours to assess cell viability. Absorbance at 450 nm was measured using a microplate reader (PerkinElmer Multimode Plate Reader; PerkinElmer, Wellesley, MA, USA). The percentage of viable cells was compared between the treated group and control groups. The obtained results are shown in FIG. 10.

As can be seen in FIG. 10, the cell viability of all test materials was 85% or more in the concentration range corresponding to 0.5 to 250 ng/mL TRP, and even at the highest concentrations of TRP (1:5:2)-15 and TRP (1:5:7)-15, the viability remained at 96.3% ± 5.39% and 98.5% ± 2.37%, respectively.

Next, to evaluate the biological activity of TRP/LDA/DA-DM, UMR-106 cells were inoculated into 96-well plates at a density of 2 × 10⁴ cells/well. After 48 hours, the cells were washed with 200 µL of PBS (pH 7.4) containing 0.5 mM phosphodiesterase inhibitor, isobutyl methylxanthine (IBMX; Sigma-Aldrich) for 10 minutes. Cells were then exposed to 100 µL of TRP (1:0:0), TRP (1:0:0)-15, TRP (1:5:2)-15, and TRP (1:5:7)-15 dissolved in PBS (pH 7.4) with 0.5 mM of IBMX corresponding to concentrations of 0.1, 0.5, 1, 5, 10, 50, and 100 nM of TRP. Then, 200 µL of supernatant from each well was centrifuged at 600 × g for 10 min at room temperature. To assess the effect of TRP/LDA/DA-DM on cyclic adenosine monophosphate (cAMP) synthesis, cAMP levels in each supernatant were quantified by competitive enzyme-linked immunosorbent assay (ELISA) (Thermo Fisher Scientific) according to the manufacturer's instructions. The obtained results are shown in FIG. 11.

As shown in FIG. 11, the biosynthesis of cAMP by TRP (1:0:0) or TRP (1:0:0)-15-activated UMR-106 cells increased in a concentration-dependent manner up to 50 nM TRP, after which the level of cAMP increased by 498% and 440%, respectively, compared to the untreated control group (i.e., 19.4 ± 1.09 pmoL/mL). It also showed similar efficacy with respect to cAMP production when compared to TRP (1:5:2)-15 or TRP (1:5:7)-15 in the concentration range of 0.1 to 50 nM. However, cells treated with TRP (1:5:2)-15 or TRP (1:5:7)-15, corresponding to 100 nM TRP, showed 139% and 165% higher cAMP levels in cell lysates than cells treated with 100 nM TRP (1:0:0), respectively, and 1.37 times and 1.63 times increase in cAMP levels, respectively, compared to cells treated with TRP (1:0:0)-15 (100 nM TRP).

### Experimental Example 6: Confirmation of oral absorption in mice

The oral absorption of TRP/LDA/DA-DM in mice was evaluated by determining the effectiveness of electrostatic complexes of TRP with LDA and DA and micellar formulations using DM. After fasting overnight, SD mice were orally administered 400 µL of TRP-Oral (1.6) (1.6 mg/kg TRP dissolved in water) or TRP (1:5:7)-15 (1.6) (equivalent to 1.6 mg/kg TRP). In addition, animals were injected subcutaneously with 200 µL of TRP-SC (0.04) (0.04 mg/kg TRP dissolved in physiological saline) to evaluate the relative oral bioavailability. Blood samples (250 µL) were collected in heparinized tubes (lithium heparin) from a cannulated femoral vein at specified time points (0, 0.167, 0.5, 1, 1.5, 2, 3, 4, 6 hours). Finally, plasma was separated by centrifugation (2500 × g, 15 min, 4°C) and stored at 80°C until analysis.

The plasma drug concentration of each sample was determined by UPLC-MS/MS. To construct a standard calibration curve, 180 µL of blank plasma was transferred to a tube, and then 10 µL of TRP at concentrations of 100, 50, 25, 10, 5, 2.5, 1, and 0 (blank) ng/mL was added to the blank plasma. To measure plasma drug concentrations after drug administration, 180 µL of mouse plasma samples at different time points were transferred to another tube, and then 10 µL hPTH (1-38) aqueous solution (100 ng/mL) (as an internal standard [IS]) was added to the TRP and blank plasma or drug-administered plasma samples. The samples were then precipitated by adding 200 µL acetonitrile containing 5% ammonium hydroxide. The precipitated sample was further diluted with 1 mL of water and centrifuged at 4°C for 15 min at 4,000 rpm. The supernatant was extracted using the solid phase extraction (SPE) method and an Oasis^{®} HLB µElution 96-well plate (Waters Corporation, Milford, MA, USA). Briefly, a 96-well plate was conditioned with 200 µL of methanol and then equilibrated with 200 µL of water. Four 350 µL aliquots of the supernatant were then added to each Oasis HLB µElution well. After the addition of standards or samples, the wells were washed with 200 µL of water containing 5% methanol. Then, TRP and IS were eluted with 25 µL of acetonitrile: water: trifluoroethanol: trifluoroacetic acid (60:34:5:1, v/v/v/v) mixture in two sequential aliquots. After further dilution of the eluate with 50 µL of water, 10 µL was injected into a UPLC system (Waters ACQUITY UPLC, Waters Corporation) equipped with an ACQUITY UPLC Peptide BEH C18 column (2.1 × 50 mm, 1.7 µm, 300 Å, Waters Corporation). The sample was placed at a low temperature of 6 °C, and the column temperature was maintained at 60 °C during the analysis. Mobile phase A is composed of 0.5% (v/v) formic acid in water, and mobile phase B is composed of acetonitrile. TRP and IS were eluted at a gradient flow rate of 0.3 mL/min, where mobile phase A was equal to 85% (initial), 85% (0.8 min), 70% (1.2 min), 70% (2.2 min), 65% (2.7 min), 10% (3.0 min), 10% (4.0 min), 85% (5 min), and 85% (5 min). For ionization, a mass spectrometer (Xevo^{™} TQ-S; Waters Corporation) was used in positive ion multiple reaction monitoring mode with electrospray voltage, source temperature, desolvation temperature, and desolvation gas flow rate of 3.0 kV, 150°C, 500°C, and 800 L/h, respectively. Quantification was performed with the transition of m/z 589.40 → 656.40 at 45 V cone voltage and 13 eV collision energy for TRP and m/z 744.20 → 855.50 at 20 V cone voltage and 20 eV collision energy for IS. The results are shown in Tables 4 and 12 and FIG. 13.

**[Table 4]**

| **Test material** | **TRP-SC (0.04)** | **TRP-Oral (1.6)** | **TRP(1: 5: 7) -15 (Example 1.6)** |
|---|---|---|---|
| Administration route | SC | Oral | Oral |
| TRP dose (mg/kg) | 0.04 | 1.6 | 1.6 |
| Tₘₐₓ (h) | 0.50 ± 0.00 | 1.30 ± 0.27 | 2.00 ± 0.00 |
| T_{1/2} (h) | 0.40 ± 0.12 | 0.72 ± 0.18 | 0.54 ± 0.05 |
| Cₘₐₓ (pg/mL) | 807 ± 135 | 155 ± 57.2 | 1190 ± 205 |
| AUCₗₐₛₜ (pg.h/mL) | 601 ± 120 | 135 ± 70.4 | 1750 ± 239 |
| AUC_{inf} (pg . h/mL) | 752 ± 26.9 | 296 ± 18.9 | 1924 ± 153 |
| Relative bioavailability (%) | 100 | 0.559 ± 0.293 | 7.27 ± 0.992 |

As shown in FIG. 12, which represents the TRP plasma concentration-time profile after SC or oral administration, the maximum plasma concentration (Cₘₐₓ) achieved after oral administration of 1.6 mg/kg of TRP alone was 155 ± 57.2 pg/mL, and the area under the plasma concentration-time curve (AUCₗₐₛₜ) was 135 ± 70.4 pgh/mL, and the oral bioavailability for SC administration was 0.559 ± 0.293%. Compared to TRP alone, oral TRP (1:5:7)-15 (equivalent to 1.6 mg/kg of TRP) increased Cₘₐₓ (1190 ± 205 pg/mL) and AUCₗₐₛₜ (1750 ± 239 pgh/mL) by 7.66 and 13.0 times, respectively, resulting in a relative oral bioavailability increase of 7.27 ± 0.992%. Time to maximum concentration (Tₘₐₓ) values were 0.5 and 2 hours after SC and oral administration of free TRP and TRP (1:5:7)-15, respectively. However, the terminal elimination half-life (T_{1/2}) after oral intake of TRP (1:5:7)-15 was 0.54 h, similar to that after SC administration (0.4 h), suggesting that TRP absorption is very delayed after oral intake. Therefore, oral TRP (1:5:7)-15 is expected to exhibit similar pulsatile action to SC TRP alone.

### Experimental Example 7: In vivo anti-osteoporotic effect of oral TRP/LDA/DA-DM in dexamethasone-induced mouse osteoporosis model

To determine the anabolic effect of orally administered TRP/LDA/DA-DM on GC-induced osteoporosis, 9-week-old female C57BL/6 mice were randomly divided into 6 groups (n = 10 per group): Control group (normal) (SC injection of normal saline once daily); Control group (DEX) (SC injection of 0.022 mg/kg DEX once daily); TRP-SC (0.02) (SC injection of 0.02 mg/kg TRP dissolved in physiological saline containing 0.022 mg/kg DEX once daily); TRP-oral (0.4) (SC injection of 0.022 mg/kg DEX dissolved in water with 0. 4 mg/kg TRP orally once daily); RP (1:5:7)-15(0.4) (once daily oral administration of TRP (1:5:7)-15 equivalent to 0.4 mg/kg TRP with SC injection of 0.022 mg/kg DEX) ; and TRP (1:5:7)-15(0.8) (once daily oral administration of TRP (1:5:7)-15 equivalent to 0.8 mg/kg TRP with SC injection of 0.022 mg/kg DEX) . Blood samples were collected for biochemical analysis 4 weeks after treatment.

In addition, femurs from 10 mice in each group were collected and fixed in 4% paraformaldehyde for bone density and histomorphometric analysis. The trabecular bone microstructure of the left femur was investigated using a Quantum GX micro-computed tomography (micro-CT) imaging system (PerkinElmer, Hopkinton, MA, USA) located at the Korea Institute of Basic Science (Gwangju, Korea). X-ray source settings included a voltage of 90 kV and a current of 88 pA, with a field of view of 10 mm (voxel size, 20 um; scan time, 4 min). Three-dimensional (3D) images were obtained with 3D Viewer, an existing software in Quantum GX, and the resolution was set to 4.5 um. After scanning, the structural parameters of the trabecular bone were analyzed using Analyze 12.0 software (Analyze Direct, Overland Park, KS, USA).

For histological observations, formalin-fixed femurs were calcified in 10% ethylenediaminetetraacetic acid (EDTA) and dehydrated by passing through a graded ethanol series. Calcified femurs were then embedded in paraffin wax, cut into 5-um-thick sections, and stained with hematoxylin and eosin (H&E). Histological sections were observed under an optical microscope (Bx41; Olympus, Tokyo, Japan), and photographs were taken focusing on the central portion of the growth plate.

The results of each experiment are shown in FIGS. 14 to 21.

As shown in FIG. 14, BMD of the total femur was significantly reduced in DEX-treated mice compared to untreated normal controls (174 ± 21.0 vs. 123 ± 7.92 mg/cm³). In addition, as can be seen in FIGS. 15 to 19, the structural parameters of trabecular bone, BV/TV, Tb.N, Tb.Th, Tb.Sp, and SIM were significantly changed in DEX-treated mice compared to the control (normal) group, with significant changes in Tb.Th (-7.98%) and SMI (-21.1%), but there was no difference in BV/TV, Tb.Sp or Tb.N. On the other hand, co-administration of TRP-SC (0.02) prevented DEX-related decrease in femoral bone mineral density, resulting in 29.4% higher BMD than the DEX group. Specifically, TRP-SC (0.02) resulted in higher parameter values in BV/TV, Tb.Th, and Tb.N by 13.2%, 45.2%, and 2.8%, respectively, and 17.4% lower parameter values in Tb.Sp than the control (DEX) group. In addition, oral administration of TRP (1:5:2)-15(0.4) or TRP (1:5:2)-15(0.8) once a day also significantly prevented BMD reduction, resulting in 36.9% and 61.1% higher BMD than the control (DEX) group, respectively, and TRP (1:5:2)-15(0.4) and TRP (1:5:2)-15(0.8) oral administration were positive for trabecular bone microarchitecture parameters. Moreover, compared to control (DEX) mice, Tb.Th was 31.3% and 28.4% higher, Tb.N was 10.5% and 14.8% higher, and Tb.Sp was 14.9% and 17.3% lower, respectively. In contrast, daily oral administration of 0.4 mg/kg of free TRP [i.e., TRP-oral (0.4)] did not produce a significant improvement in the BMD loss observed in TRP (1:5:2)-15(0.4) mice.

As can be seen in FIG. 20, an increase in the trabecular bone parameter can be easily observed in the 3D micro-CT image of the femur. In contrast to the spontaneous bone growth observed in the control (normal) group, deterioration of cancellous bone structure was observed in DEX-induced osteoporotic mice. However, the TRP-SC (0.02), TRP (1:5:2)-15 (0.4), and TRP (1:5:2)-15 (0.8) groups showed denser trabecular bone microarchitecture in the proximal femoral region compared to the control (DEX) group and TRP-Oral (0.4) group.

As can be seen in FIG. 21, normal control mice exhibited dense and homogeneous trabeculae in contrast to DEX-treated mice, while TRP-SC (0.02) and oral TRP (1:5:2)-15 (0.4 or 0.8) groups exhibited increased structural integrity and denser trabecular bone microarchitecture than control (DEX) group and TRP-Oral (0.4) groups.

### Experimental Example 8: Biochemical analysis

After the final treatment once daily, serum samples were collected from mouse whole blood by centrifugation at 2000 ×g for 20 minutes. Then, the serum levels of osteocalcin, procollagen I N-terminal pro-peptide (PINP), and C-terminal cross-linked telopeptide of type I collagen (CTx) in each sample were determined according to the manufacturer's instructions using the Mouse Osteocalcin ELISA kit (LifeSpan BioSciences, Inc., Seattle, WA, USA), Rat/Mouse PINP EIA kit (Immunodiagnostic Systems Holdings Ltd., Tyne & Wear, UK), and RatLaps^{™} CTx-I EIA (Immunodiagnostic Systems Holdings Ltd.) was used to confirm each. In addition, the serum calcium level was confirmed through a colorimetric assay at 560 nm (Sigma-Aldrich). The results are shown in FIGS. 22 to 25.

As can be seen in FIG. 22, after 4 weeks of DEX treatment, there was no significant change in the serum osteocalcin level compared to the control (normal) group. However, when 0.02 mg/kg of TRP was subcutaneously injected once a day or TRP (1:5:7)-15 was orally administered for 4 weeks, serum osteocalcin was significantly increased. That is, the serum osteocalcin level of TRP-SC (0.02) was 18.2%, 28.2%, and 9.68% higher than that of the control group (normal), control group (DEX), and TRP-oral (0.4) group, respectively, and the osteocalcin level in the TRP (1:5:7)-15(0.4) and TRP (1:5:7)-15(0.8) groups increased by 34.9% and 46.2%, respectively, compared to the control group (DEX).

On the other hand, as can be seen in FIG. 23, after DEX treatment, serum PINP was significantly reduced by 26.0% compared to the control (normal) group. TRP-SC (0.02) showed 1.19 times higher PINP compared to the control (DEX) group, and daily oral administration of TRP(1:5:7)-15, equivalent to 0.4 or 0.8 mg/kg of TRP, increased PINP secretion by 18.6% and 19.0%, respectively, compared to DEX-treated mice only. On the other hand, it was confirmed that oral absorption of TRP from TRP (1:5:7)-15 was enhanced because serum PINP was not recovered after oral administration of TRP-oral (0.4) compared to the control group (DEX) .

From FIG. 24, serum CTx was significantly increased after 4 weeks of DEX treatment, resulting in a 48.9% increase in serum CTx when compared to the control (normal) group, indicating severe bone resorption. On the other hand, CTx levels did not increase significantly in the TRP treatment group, and oral TRP (1:5:7)-15 (0.4) or TRP (1:5:7)-15 (0.8) inhibited the decrease in serum CTx by 25.3% and 23.8%, respectively, confirming that oral treatment with TRP (1:5:7)-15 induced more bone formation than bone resorption.

Serum calcium levels were measured to determine whether TRP treatment caused a rapid increase in bone resorption in mice, and as shown in FIG. 25, administration of DEX significantly downregulated serum calcium levels compared to the control (normal) group. In contrast, all TRP-treated groups exhibited significantly higher serum calcium concentrations compared to DEX-induced osteoporotic and normal control mice. In addition, SC injection of 0.02 mg/kg TRP or oral administration of TRP (1:5:7)-15, corresponding to 0.4 mg/kg TRP, increased serum calcium levels to a maximum of 21.4% and 25.7%, respectively, compared to the control (normal) group at 6 hours and then decreased to the same level as the control (normal) group. However, mice treated with oral TRP (1:5:7)-15(0.8) showed 21.2% to 42.5% higher serum calcium levels than the control (normal) group. Putting the results all together, these observations demonstrate that once-daily oral administration of TRP (1:5:7)-15 stimulates bone turnover in DEX-induced osteoporotic mice primarily by stimulating bone formation.

## Claims

1. An oral pharmaceutical composition for prevention and treatment of osteoporosis, the composition comprising:
teriparatide;
at least one of L-lysine-linked deoxycholic acid and deoxycholic acid; and
an ion-binding complex composed of an alkyl glycoside-based surfactant.

2. The composition of claim 1, wherein the alkyl glycoside-based surfactant is at least one of n-dodecyl-β-D-maltoside and n-octyl-β-D-glucopyranoside.

3. The composition of claim 1, wherein the lysine-linked deoxycholic acid is contained in a range of 1 to 7 moles per 1 mole of yhe teriparatide.

4. The composition of claim 1, wherein the deoxycholic acid is contained in a range of 1 to 15 moles per 1 mole of teriparatide.

5. The composition of claim 1, wherein the alkyl glycoside-based surfactant and the teriparatide are contained in a weight ratio of 1:1 to 1:50.

6. A method for preparing an oral pharmaceutical composition for prevention and treatment of osteoporosis, the method comprising:
a first step of preparing a teriparatide solution, a deoxycholic acid solution, a lysine-linked deoxycholic acid solution, and an alkyl glycoside-based surfactant solution;
a second step of mixing the alkyl glycoside-based surfactant solution with the teriparatide solution prepared in the first step;
a third step of mixing the lysine-linked deoxycholic acid solution with the mixed solution obtained in the second step; and
a fourth step of mixing the deoxycholic acid solution with the mixed solution obtained in the third step.

7. The method of claim 6, wherein each of the teriparatide solution, the deoxycholic acid solution, and the lysine-linked deoxycholic acid solution has a pH value in a range of 3 to 8.

8. The method of claim 6, wherein in the second step, the alkyl glycoside-based surfactant and the teriparatide are in a weight ratio of 1:1 to 1:50.

9. The method of claim 6, wherein in the third step, the lysine-linked deoxycholic acid is contained in a range of 1 to 7 moles per 1 mole of teriparatide.

10. The method of claim 6, wherein in the fourth step, the deoxycholic acid is contained in a range of 1 to 15 moles per 1 mole of teriparatide.

11. The method of claim 6, further comprising
adding mannitol and sucrose to the mixture of step 4, followed by drying.
